# EUROPÄISCHE PATENTSCHRIFT

(11) **EP 1 198 254 B1**
(45) Veröffentlichungstag und Bekanntmachung des Hinweises auf die Patenterteilung: **15.08.2007**
(21) Anmeldenummer: 00943777.3
(22) Anmeldetag: 07.06.2000
(51) Int. Cl.: A61K 47/48

(54) **TRÄGER-PHARMAKA-KONJUGATE**
CARRIER-DRUG CONJUGATE
CONJUGUE EXCIPIENT-PRODUIT PHARMACEUTIQUE

(30) Priorität: 10.06.1999 DE 19926475
(43) Veröffentlichungstag der Anmeldung: 24.04.2002
(73) Patentinhaber: KTB Tumorforschungsgesellschaft mbH, 79106 Freiburg (DE)
(72) Erfinder: KRATZ, Felix, 79241 Ihringen (DE)
(74) Vertreter: Müller-Boré & Partner Patentanwälte
(86) Internationale Anmeldenummer: PCT/EP2000/005254
(87) Internationale Veröffentlichungsnummer: WO 2000/076550

(56) Entgegenhaltungen:
- WO-A-00/76551
- WO-A-93/08842
- DE-A- 19 636 889
- KRATZ F ET AL: "PREPARATION, CHARACTERIZATION AND IN VITRO EFFICACY OF ALBUMIN CONJUGATES OF DOXORUBICIN" BIOLOGICAL & PHARMACEUTICAL BULLETIN (OF JAPAN),JP,PHARMACEUTICAL SOCIETY OF JAPAN, Bd. 21, Nr. 1, 1998, Seiten 56-61, XP000738275 ISSN: 0918-6158
- A. TROUET ET AL.: "A covalent linkage between daunorubicin and proteins that is stable in serum and reversible by lysosomal hydrolases, as required for a lysosomotropc drug-carrier conjugate; In vitro and in vivo studies." PROCEEDINGS OF THE NATIONAL ACADEMY OF SCIENCES OF USA, Bd. 79, Januar 1982 (1982-01), Seiten 626-629, XP002162367 WASHINGTON US
- S. NETZEL-ARNETT: "Comparitive sequence specificities of human 72- and 92-kDa gelatinases (type iv Collagenases) and PUMP (Matrilysin)" BIOCHEMISTRY, Bd. 32, Nr. 25, 1993, Seiten 6427-6432, XP002162368 EASTON, PA US in der Anmeldung erwähnt
- M. NICHIFOR ET AL.: "Macromolecular prodrugs of 5-fluorouracil. 2: Enzymatic degradation." JOURNAL OF CONTROLLED RELEASE, Bd. 39, 1996, Seiten 79-92, XP002162369 AMSTERDAM NL
- G. M. DUBOWCHIK ET AL. : "Cathepsin B-sensitive dipeptide prodrugs. 1. A model study of structural requirements for efficient release of doxorubicin." BIOORG MED CHEM LETT., Bd. 8, Nr. 23, Dezember 1998 (1998-12), Seiten 3341-3346, XP002162370
- Derossi et al. (1998) Trends Cell Biol. 8, 84-87.
- Narazaki et al. (1997) BBA 1338, 275-281.
- Kratz et al (1999) Crit. Rev. Ther. Drug. Carrier Syst. 16, 245-288.
- Firestone et al. (1996) J. Controll. Rel. 39, 251-259.
- Willner et al. (1993) Bioconjugate Chem. 4, 521-527.
- Derossi et al: (1998) Trends Cell Biol. 8, 84-87.
- Narazaki et al: (1997) BBA 1338, 275-281.
- Kratz et al: (1999) Crit. Rev. Ther. Drug. Carrier Syst. 16, 245-288.
- Firestone et al: (1996) J. Controll. Rel. 39, 251-259.
- Willner et al: (1993) Bioconjugate Chem. 4, 521-527.
- Yasuzawa & Tomer (1997) Bioconjugate Chem. 8, 391-399.
- Burton et al. (1989) Eur. J. Biochem. 179, 379-387.
- Kratz et al. (2002) J. Med. Chem. 45, 5523-5533 + Supporting info.
- Kratz et al. (2001) Bioorg. Med. Chem. Lett. 11, 2001-2006.
- Stehle et al. (1997) Anti-Cancer Drugs 8, 677-685.

## Beschreibung

Die vorliegende Erfindung betrifft Träger-Pharmaka-Konjugate, sowie Verfahren zu deren Herstellung und Arzneimittel, welche die Konjugate enthalten.

Der Großteil der zur Zeit eingesetzten Pharmaka sind niedermolekulare Verbindungen und weisen nach systemischer Applikation eine hohe Plasma- sowie Gesamtclearance auf. Desweiteren dringen sie aufgrund von Diffusionsvorgängen in die Gewebestrukturen des Körpers ein und weisen in der Regel eine gleichmäßige Bioverteilung auf. Beide Eigenschaften führen dazu, daß nur geringe Mengen des Pharmakons den Wirkort erreichen und das Pharmakon aufgrund seiner Verteilung auf das gesunde Gewebe des Körpers Nebenwirkungen hervorruft. Diese Nachteile sind besonders bei solchen Pharmaka ausgeprägt, die ein hohes zytotoxisches Potential besitzen, wie etwa Zytostatika oder Immunsuppressiva.

Aus diesem Grund wird nach neuen Derivaten bzw. Formulierungen gesucht, die eine selektivere Therapie ermöglichen. Zu diesem Zwecke werden Chemoimmunokonjugate bzw. Protein- oder Polymerkonjugate, bestehend aus einer geeigneten Trägersubstanz und einem Pharmakon, entwickelt.

Zum Stand der Technik auf diesem Gebiet sind Polymerkonjugate zu nennen, bei denen Zytostatika an Serumproteine, Antikörper, Wachstumsfaktoren, hormon- bzw. peptidähnliche Strukturen oder an synthetische Polymere gekoppelt sind (Mägerstädt, M.: Antibody Conjugates and Malignant Disease, Library of Congress 1990; Seymour, L.W. Crit. Rev. Ther. Drug Carrier Sys. (1992), 9, 135-187; Maeda, H.; Matsumura, Y. Crit. Rev. Ther. Drug Carrier Sys. (1989), 6, 193-210).

< Kratz et al. (Biol. Pharm. Bull. 21(1), 56-61, 1998) beschreibt Albumin-Doxorubicin-Konjugate, wobei in Albumin als Träger Thiolgruppen eingeführt werden. Firestone et al. (J. Con. Release 39, 251-259, 1996) und Willner et al. (Bioconjugate Chem. 4, 521-527,1993) beschreiben Doxorubicin-Antikörper-Konjugate. >

Aus DE-A-41 22 210 sind Konjugate tumoraktiver Verbindungen mit Albumin bekannt, wobei die tumoraktive Verbindung mit N-Hydroxysuccinimid und Carbodiimid aktiviert und das so erhaltene Gemisch direkt an das Trägerprotein gekoppelt wird. Nachteile dieser Konjugate liegen u.a. darin, daß sie nicht in der erforderlichen hohen Reinheit gewonnen werden können, die native Struktur des Albumins aufgrund der Herstellungsverfahren oft nicht erhalten bleibt und das stöchiometrische Verhältnis von Pharmakon zu Albumin nicht konstant und schlecht reproduzierbar ist. Desweiteren ermöglichen es diese Konjugate nicht, daß sie in geeigneter Weise im Zietgewebe bzw. in den Zielzellen freigesetzt werden können.

Daher liegt der vorliegenden Erfindung die Aufgabe zugrunde, neue Träger-Pharmaka-Konjugate bereitzustellen, welche die Nachteile der im Stand der Technik bekannten Konjugate überwinden.

Diese Aufgabe wird durch die in den Ansprüchen gekennzeichneten Ausführungsformen der vorliegenden Erfindung gelöst.

Insbesondere wird ein Träger-Pharmakon-Konjugat bereitgestellt, umfassend natives oder rekombinantes Albumin als Träger und ein Pharmakon, enthaltend eine pharmazeutisch und/oder diagnostisch aktive Substanz, ein Spacermolekül und eine thiolbindende Gruppe, wobei pro Mol Cystein-34-Rest mehr als 0,7 Mol, vorzugsweise mindestens 0,9 Mol, Pharmakon über die thiolbindende Gruppe pro Mol Albumin an Cystein-34 gebunden sind. Der Ausdruck "pharmazeutisch aktive Substanz" bedeutet, daß die betreffende Substanz entweder selbst oder nach ihrer Umsetzung durch den Stoffwechel im jeweiligen Organismus eine pharmakologische Wirkung hervorruft und umfaßt somit auch die sich durch diese Umsetzungen ergebenden Derivate. Selbstverständlich kann die pharmazeutisch aktive Substanz ein einzelnes (beispielsweise nur als Zytostatikum) oder ein breites (beispielsweise als Zytostatikum und als Antiphlogistikum usw.) pharmakologisches Wirkspektrum aufweisen. Der Ausdruck "diagnostisch wirksame Substanz" bedeutet, daß die betreffende Substanz mittels geeigneter chemischer und/oder physikalischer Meßmethoden im Organismus oder Teilen davon wie beispielsweise Zellen und/oder Flüssigkeiten wie beispielsweise dem Serum nachweisbar, vorzugsweise auch quantifizierbar ist.

Die Freisetzung der pharmazeutisch wirksamen Substanz ist bevorzugt, da in der Regel der niedermolekulare Wirkstoff mit dem Zielmolekül wechselwirken muß, um seine pharmakologische Wirksamkeit zu entfalten. Bei diagnostisch wirksamen Substanzen ist in der Regel eine Freisetzung des an das Albumin gebundenen Diagnostikums nicht erforderlich, kann jedoch vorhanden sein. Erfindungsgemäß kann deshalb insbesondere eine diagnostisch wirksame Sugstanz zusätz-lich über eine im Körper nicht spaltbare Bindung an das Spacermolekül oder direkt an die Trägermolekül-bindende Gruppe gebunden sein.

Das Pharmakon bzw. das Pharmakonderivat im erfindungsgemäßen Konjugat läßt sich beispielsweise durch das folgende Schema darstellen (AS, pharmazeutisch und/oder diagnostisch aktive Substanz; SM, Spacermolekül; TG, thiolbindende Gruppe):

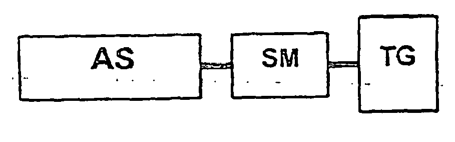

Das erfindungsgemäße Konjugat stellt eine Transport- und/oder Depotform der pharmazeutisch und/oder diagnostisch aktiven Substanz dar, die so gezielt bzw. in dosierter Form die Zielzellen bzw. das Zielgewebe des Pharmakons erreicht. Gegenüber den bisher bekannten Konjugaten können die Konjugate der vorliegenden Erfindung in einer höheren Reinheit gewonnen werden, die native Struktur des Trägers bleibt erhalten und das stöchiometrische Verhältnis von Pharmakon zu Träger ist konstant und reproduzierbar.

Gegenüber den in DE-A-41 22 210 beschriebenen Albumin-Zytostatika-Konjugaten besitzt das erfindungsgemäße Konjugat weiterhin den Vorteil, daß zwischen der pharmazeutisch und/oder diagnostisch aktiven Substanz und der thiolbindenden Gruppe ein Spacermolekül vorhanden ist, das so maßgeschneidert' ist, daß die pharmazeutisch und/oder diagnostisch aktive Substanz oder ein entsprechendes aktives Derivat hiervon im Zielgewebe bzw. in den Zielzellen hydrolytisch und/oder pH-abhängig und/oder enzymatisch freigesetzt werden kann.

Albumin bzw. dessen Pharmaka-Konjugate weisen eine ausgesprochen lange Halbwertszeit im systemischen Kreislauf auf (bis zu 19 Tage - Peters, T. Jr. (1985): Serum albumin. Adv. Protein. Chem. 37, 161-245). Aufgrund einer erhöhten Permeabilität von Gefäßwänden des malignen, infizierten bzw. entzündeten Gewebes für Makromoleküle gelangt Serumalbumin bevorzugt in das Zielgewebe (Maeda, H.; Matsumura, Y. Crit. Rev. Ther. Drug Carrier Sys. (1989), 6, 193-210). Dadurch kann ein an einen Träger, z.B. Albumin, gekoppelter Wirkstoff gezielter den Wirkort erreichen. Desweiteren verhindert das erfindungsgemäße Albumin-Pharmakon-Konjugat, das die pharmazeutisch und/oder diagnostisch aktive Substanz in gesunde Gewebestrukturen des Körpers diffundiert oder über die Niere eliminiert wird bzw. diese in dem Maße schädigt wie die nicht gebundene pharmazeutisch und/oder diagnostisch aktive Substanz. Dadurch wird das pharmakokinetische Profil der pharmazeutisch und/oder diagnostisch aktiven Substanz verändert und verbessert, da die Wirkung der pharmazeutisch und/oder diagnostisch aktiven Substanz durch eine Anreicherung am Wirkort erhöht wird und gleichzeitig die toxischen Wirkungen auf gesunde Systeme des Körpers verringert werden.

Das Konjugat der vorliegenden Erfindung besitzt eine ausgezeichnete Wasserlöslichkeit. Desweiteren zeigt das erfindungsgemäße Konjugat *in vivo* beispielsweise eine verbesserte antitumorale Wirksamkeit gegenüber der ungebundenen pharmazeutisch und/oder diagnostisch aktiven Substanz.

Gemäß einer bevorzugten Ausführungsform des erfindungsgemäßen Konjugats ist das Spacermolekül und/oder die Verknüpfung zwischen der pharmazeutisch und/oder diagnostisch aktiven Substanz und dem Spacermolekül und/oder die Verknüpfung zwischen der thiolbindenden Gruppe und dem Spacermolekül hydrolytisch und/oder pH-abhängig und/oder enzymatisch spaltbar. Vorzugsweise enthält das Spacermolekül und/oder die Verknüpfung zwischen der pharmazeutisch und/oder diagnostisch aktiven Substanz und dem Spacermolekül und/oder die Verknüpfung zwischen der thiolbindenden Gruppe und dem Spacermolekül mindestens eine säurelabile Bindung. Beispiele säurelabiler Bindungen sind Ester-, Acetal-, Ketal-, Imin-, Hydrazon, Carboxylhydrazon-, Sulfonylhydrazon und eine Tritylgruppe enthaltende Bindungen. Bindungen, die durch Hydrolyse unter Freisetzung der pharmazeutisch und/oder diagnostisch aktiven Substanz gespalten werden, sind beispielsweise Esterbindungen oder Metallkomplexverbindungen, wie sie bei Platin-Dicarboxylat-Komplexen vorliegen, wobei ein Diamindiaquo-Platin(II)-Komplex freigesetzt wird. Beispiele für im Körper nicht spaltbare Bindungen, die beispielsweise bei der Verknüpfung zu einer diagnostisch wirksamen Substanz vorliegen können, sind Amidbindungen, gesättigte und ungesättigte Kohlenstoff-Kohlenstoff-Bindungen oder Bindungen zwischen Kohlenstoff und einem Heteroatom, -C-X-, wobei X vorzugsweise O N, S oder P ist.

Gemäß einer weiteren Ausführungsform des erfindungsgemäßen Konjugats enthält das Spacermolekül und/oder die Verknüpfung zwischen der pharmazeutisch und/oder diagnostisch aktiven Substanz und dem Spacermolekül und/oder die Verknüpfung zwischen der thiolbindenden Gruppe und dem Spacermolekül mindestens eine Peptidbindung. Vorzugsweise liegt die Peptidbindung innerhalb einer Peptidsequenz vor, welche mindestens eine Spaltsequenz einer Protease enthält. Daher kann die mindestens eine Peptidbindung durch Einfügen einer Peptidsequenz in das Spacermolekül und/oder in die Verknüpfung zwischen der pharmazeutisch und/oder diagnostisch aktiven Substanz und dem Spacermolekül und/oder in die Verknüpfung zwischen der thiolbindenden Gruppe und dem Spacermolekül realisiert werden, d.h. die jeweilige Verknüpfung ist eine Peptidbindung, und besteht vorzugsweise aus etwa 1 bis 30 Aminosäuren. Die Peptidsequenz ist dabei vorzugsweise auf die Substratspezifität bestimmter körpereigener Enzyme oder von Enzymen zugeschnitten, die in Mikroorganismen vorkommen bzw. von diesen gebildet werden. Dadurch wird die Peptidsequenz oder ein Teil dieser Sequenz im Körper von den Enzymen erkannt und das Peptid gespalten.

Die Enzyme sind beispielsweise Proteasen und Peptidasen, z.B. Matrix-Metallo-proteasen (MMP), Cysteinproteasen, Serinproteasen und Plasminaktivatoren, die bei Erkrankungen wie rheumatoider Arthritis oder Krebs verstärkt gebildet oder aktiviert sind, was zum exzessiven Gewebeabbau, zu Entzündungen und zur Metastasierung führt. Targetenzyme sind insbesondere MMP 2, MMP 3 und MMP 9, die als Proteasen bei den genannten pathologischen Prozessen beteiligt sind (Vassalli, J., Pepper, M.S. (1994), Nature 370, 14-15, Brown, P.D. (1995), Advan. Enzyme Regul. 35, 291-301) .

Weitere Proteasen, die Targetenzyme für Konjugate der vorliegenden Erfindung darstellen, sind Cathepsine, insbesondere Cathepsin B und H, die als Schlüsselenzyme bei entzündlichen und malignen Erkrankungen identifiziert worden sind (T. T. Lah et al. (1998), Biol. Chem. 379, 125-301).

Gemäß einer weiteren Ausführungsform des erfindungsgemäßen Konjugats enthält das Spacermolekül und/oder die Verknüpfung zwischen der pharmazeutisch und/oder diagnostisch aktiven Substanz und dem Spacermolekül und/oder die Verknüpfung zwischen der thiolbindenden Gruppe und dem Spacermolekül mindestens eine Bindung, die enzymatisch spaltbar ist, aber nicht aus einer Peptidbindung besteht. Beispiele sind Carbamatbindungen, bei denen durch Spaltung mit krankheitsspezifischen Enzymen, z.B. Glutathion-S-Transferasen, Glucuronidasen, Galactosidasen, der Wirkstoff oder ein Wirkstoffderivat freigesetzt wird. Es ist auch ohne weiteres möglich, dass eine enzymatisch spaltbare Bindung aus einer Peptidsequenz und einer der vorstehend genannten Bindungen, die keine Peptidbindung ist, aufgebaut ist.

Alle genannten Bindungstypen - hydrolytisch spaltbare Bindung, säurelabile Bindung, Peptidbindung, enzymatisch spaltbare Bindung, die keine Peptidbindung enthält, und eine Bindung, die aus einer Peptidsequenz und einer nicht-Peptidbindung aufgebaut ist - gewährleisten, daß die pharmazeutisch und/oder diagnostisch wirksame Substanz oder ein entsprechend aktives Derivat am Wirkort extrazellulär und/oder intrazellulär gespalten wird und die Substanz seine pharmazeutische und/oder diagnostische Wirkung entfalten kann.

Gemäß einer bevorzugten Ausführungsform ist die pharmazeutisch aktive Substanz ein Zytostatikum, ein Zytokin, ein Immunsuppressivum, ein Antirheumatikum, ein Antiphlogistikum, ein Antibiotikum, ein Analgetikum, ein Virostatikum oder ein Antimyotikum. Besonders geeignete Zytostatika der Konjugate der vorliegenden Erfindung sind die N-Nitrosoharnstoffe wie Nimustin, die Anthrazykline Doxorubicin, Daunorubicin, Epirubicin, Idarubicin, Mitoxantron und Ametantron sowie verwandte Derivate, die Alkylantien Chlorambucil, Bendamustin, Melphalan und Oxazaphosphorine sowie verwandte Derivate, die Antimetabolite, beispielsweise Purin- oder Pyrimidinantagonisten und Folsäureantagonisten wie Methotrexat, 5-Fluorouracil, 5'-Desoxy-5-fluorouridin und Thioguanin sowie verwandte Derivate, die Taxane Paclitaxel und Docetaxel sowie verwandte Derivate, die Camptothecine Topotecan, Irinotecan, 9-Aminocamptothecin und Camptothecin sowie verwandte Derivate, die Podophyllotoxinderivate Etoposid, Teniposid und Mitopodozid sowie verwandte Derivate, die Vinca-Alkaloide Vinblastin, Vincristin, Vindesin und Vinorelbin sowie verwandte Derivate, Calicheamicine, Maytansinoide und cis-konfigurierte Platin(II)-Komplexverbindungen der allgemeinen Formeln I bis XII: 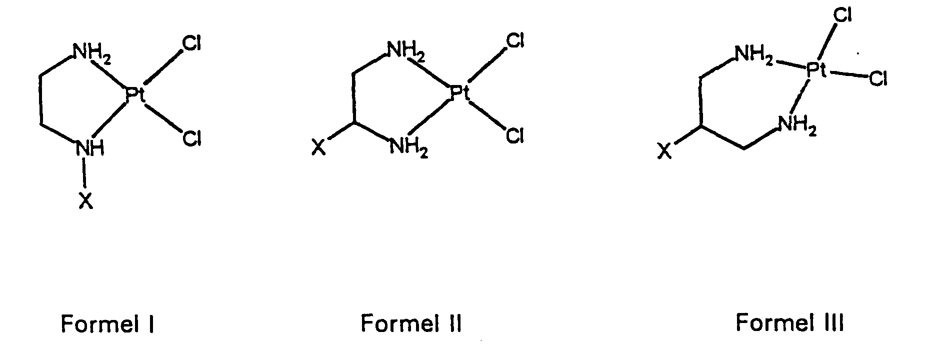 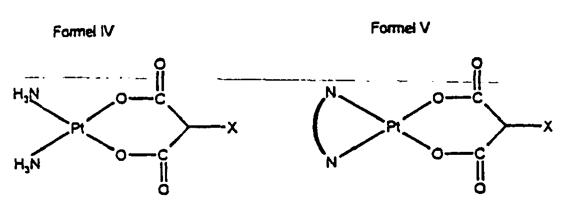 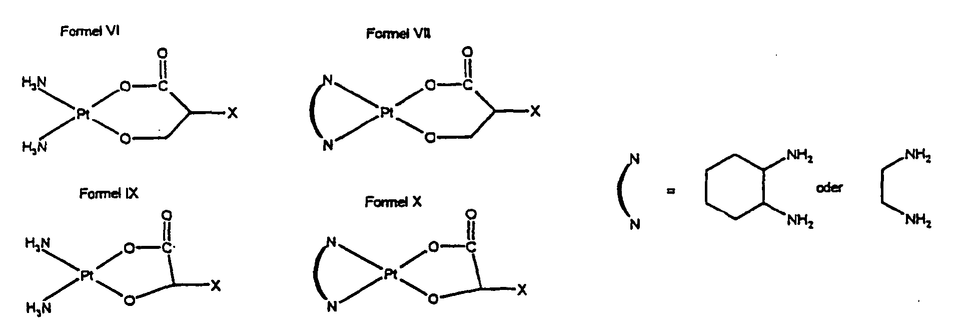 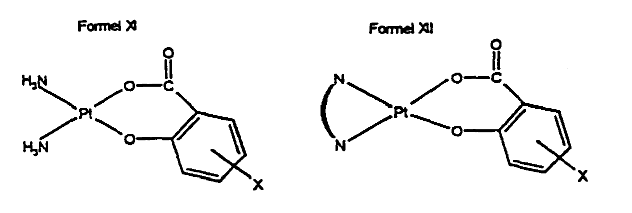 wobei X das Spacermolekül oder die thiolbindende Gruppe ist.

Besonders geeignete Zytokine in Konjugaten der vorliegenden Erfindung sind beispielsweise Interleukin 2, Interferon α-2a, Interferon α-2b, Interferon β-1a, Interferon β-1b, Interferon γ-1b und verwandte Derivate. Die verwendeten Zytokine sind i.d.R. gentechnisch hergestellte Arzneimittel.

Besonders geeignete lmmunsuppresiva in Konjugaten der vorliegenden Erfindung sind beispielsweise Cyclosporin A, FK 506 und verwandte Derivate.

Besonders geeignete Antirheumatika in Konjugaten der vorliegenden Erfindung sind beispielsweise Methotrexat, Sulfasalazin, Chloroquin und verwandte Derivate.

Besonders geeignete Antiphlogistika und/oder Analgetika in Konjugaten der vorliegenden Erfindung sind beispielsweise Salicylsäurederivate, wie etwa Acetylsalicylsäure und verwandte Derivate, Pharmaka-Derivate, die eine Essig-oder Propionsäuregruppe aufweisen, wie etwa Diclofenac bzw. Indometacin oder Ibuprofen bzw. Naproxen, und Aminophenolderivate, wie etwa Paracetamol.

Besonders geeignete Antimyotika in Konjugaten der vorliegenden Erfindung sind beispielsweise Amphotericin B und verwandte Derivate.

Bevorzugte Virostatika in Konjugaten der vorliegenden Erfindung sind beispielsweise Nukleosidanaloga, wie Aciclovir, Ganciclovir, Idoxuridin, Ribavirin, Vidaribin, Zidovudin, Didanosin und 2',3'-Didesoxycytidin (ddC) und verwandte Derivate sowie Amantadin.

Bevorzugte Antibiotika im erfindungsgemäßen Konjugat sind Sulfonamide, beispielsweise Sulanilamid, Sulfacarbamid und Sulfametoxydiazin und verwandte Derivate, Penicelline, beispielsweise 6-Aminopenicillansäure, Penicellin G sowie Penicellin V und verwandte Derivate, Isoxazoylpenicelline, wie Oxacillin, Cloxacillin und Flucloxacillin sowie verwandte Derivate, α-substituierte Benzylpenicelline, wie Ampicillin, Carbenicillin, Pivampicillin, Amoxicillin und verwandte Derivate, Acylaminopenicelline, beispielsweise Mezlocillin, Azlocillin, Piperacillin, Apalicillin und verwandte Derviate, Amidinopenicilline, beispielsweise Mecillinam, atypische β-Lactame, wie Imipenam und Aztreonam, Cephalosporine, beispielsweise Cefalexin, Cefradin, Cefaclor, Cefadroxil, Cefixim, Cefpodoxim, Cefazolin, Cefazedon, Cefuroxim, Cefamandol, Cefotiam, Cefoxitin, Cefotetan, Cefmetazol, Latamoxef, Cefotaxim, Ceftriaxon, Ceftizoxim, Cefmonoxim, Ceftazidim, Cefsulodin und Cefoperazon sowie verwandte Derivate, Tetracycline, wie Tetracyclin, Chlortetracyclin, Oxytetracyclin, Demeclocyclin, Rolitetracyclin, Doxycyclin, Minocyclin und verwandte Derivate, Chloramphenicole, wie Chlor-amphenicol und Thiamphenicol sowie verwandte Derivate, Gyrasehemmstoffe, beispielsweise Nalixidinsäure, Pipemidsäure, Norfloxacin, Ofloxacin, Ciprofloxacin und Enoxacin sowie verwandte Derivate, und Tuberkulosemittel, wie Iso-niazid und verwandte Derivate.

Selbstverständlich kann im erfindungsgemäßen Konjugat pro Mol eine einzelne Pharmakonspezies (beispielsweise ein Pharmakon mit einem Zytostatikum als pharmazeutisch aktiver Substanz) oder unterschiedliche Pharmakonspezies (beispielsweise mehrere unterschiedliche Zytostatika oder ein Zytostatikum und ein Antiphlogistikum usw. als pharmazeutisch aktive Substanz) gebunden vorliegen.

Gemäß einer weiteren bevorzugten Ausführungsform des erfindungsgemäßen Konjugats umfaßt das Spacermolekül einen substituierten oder unsubstituierten, verzweigt- oder unverzweigtkettigen aliphatischen Alkylrest mit 1 bis 12 Kohlenstoffatomen und/oder mindestens einen substituierten oder unsubstituierten Arylrest und/oder einen aliphatischen Kohlenstoffring mit 3 bis 12 Kohlenstoffatomen. Der aliphatische Alkylrest enthält vorzugsweise 1 bis 20 Kohlenstoffatome, die teilweise durch Sauerstoffatome ersetzt sein können, um beispielsweise die Wasserlöslichkeit zu erhöhen, wobei sich solche Reste vorzugsweise von einer Oligoethylenoxid- oder -propylenoxidkette ableiten. Besonders geeignete Reste, die von Oligoethylenoxid- oder -propylenoxidketten abgeleitet sind, umfassen beispielsweise Di-ethylenglycol-, Triethylenglycol- und Dipropylenglycolketten. Ein bevorzugter Arylrest ist ein unsubstituierter oder substituierter Phenylrest, bei welchem ebenfalls ein oder mehrere Kohlenstoffatome durch Heteroatome ersetzt sein können. Bevorzugte Substituenten des aliphatischen Alkylrests bzw. des Arylrests sind hydrophile Gruppen, wie Sulfonsäure-, Aminoalkyl- und Hydroxygruppen.

Bevorzugte diagnostisch wirksame Substanzen des erfindungsgemäßen Konjugats enthalten beispielsweise ein oder mehrere Radionuklide, ein oder mehrere Radionuklide umfassende, vorzugsweise solche Radionuklide komplexierende Liganden, ein oder mehrere Positronenstrahler, ein oder mehrere NMR-Kontrastmittel, eine oder mehrere fluoreszierende Verbindung(en) oder ein oder mehrere Kontrastmittel im nahen IR-Bereich.

In einer weiteren bevorzugten Ausführungsform des erfindungsgemäßen Konjugats umfaßt die thiolbindende Gruppe eine Maleinimidgruppe, eine Halogenacetamidgruppe, eine Halogenacetatgruppe, eine Pyridyldithio-Gruppe, eine Vinylcarbonylgruppe, eine Aziridingruppe, eine Disulfidgruppe oder eine Acetylengruppe, die gegebenenfalls substituiert sind.

Das Pharmakon oder Pharmakaderivat der erfindungsgemäßen Konjugate kann je nach der vorliegenden funktionellen Gruppe gemäß einer der folgenden allgemeinen Beschreibungen hergestellt werden.

Pharmaka oder Pharmakaderivate der erfindungsgemäßen Konjugate, die eine HOOC-Gruppe besitzen, können beispielsweise folgendermaßen derivatisiert werden:

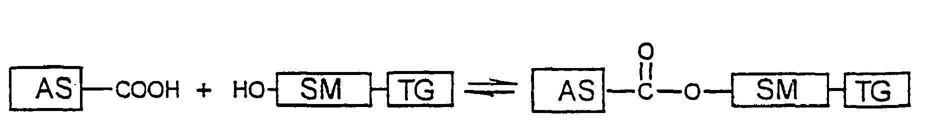

Die Veresterung erfolgt dabei durch im Stand der Technik bekannte Verfahren.

Es ist weiterhin möglich, die HOOC-Gruppe in eine Hydrazidgruppe zu überführen, z.B. durch Umsetzen mit tert.-Alkylcarbazaten und anschließende Spaltung mit Säuren (beschrieben in DE-A-196 36 889), und das eine Hydrazidgruppe aufweisende Pharmakon mit einer eine Carbonylkomponente enthaltenden Gruppe, bestehend aus der thiolbindenden Gruppe und dem Spacermolekül, umzusetzen, wie u.a. in DE-A-196 36 889 beschrieben ist:

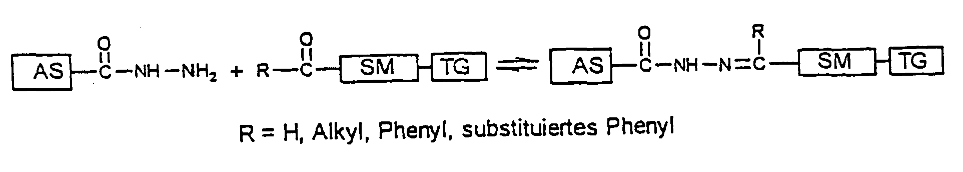

Pharmaka oder Pharmakaderivate der erfindungsgemäßen Konjugate, die eine H₂N-Gruppe besitzen, können beispielsweise folgendermaßen derivatisiert werden:

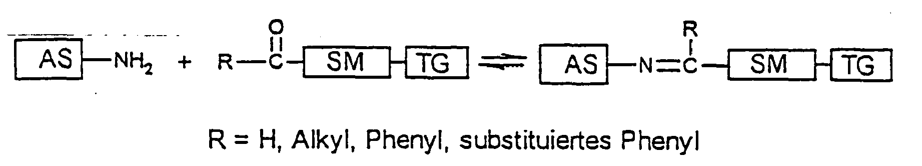

Die Reaktion zu den lminderivaten erfolgt dabei durch im Stand der Technik bekannte Verfahren.

Pharmaka oder Pharmakaderivate der erfindungsgemäßen Konjugate, die eine HO-Gruppe besitzen, können beispielsweise folgendermaßen derivatisiert werden:

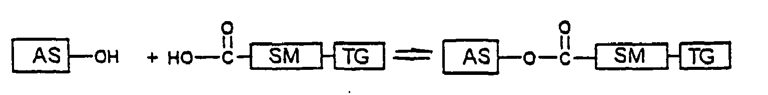

Die Veresterung erfolgt dabei durch im Stand der Technik bekannte Verfahren.

Pharmaka oder Pharmakaderivate der erfindungsgemäßen Konjugate, die eine Carbonylkomponente besitzen, können beispielsweise folgendermaßen derivatisiert werden: 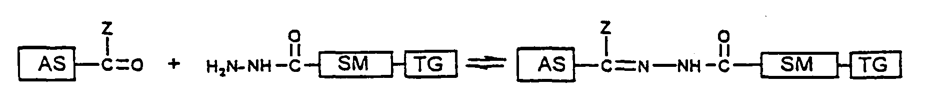 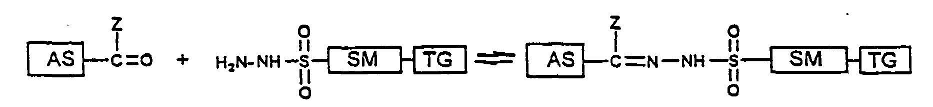 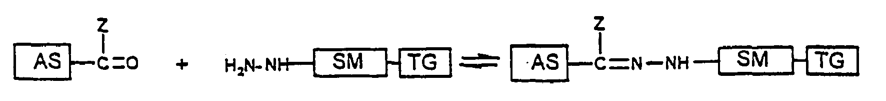 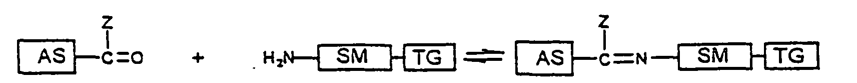 Z = chemische Gruppe der pharmazeutisch und/oder diagnostisch aktiven Substanz

Die Umsetzung zu den Carboxyhydrazon-, Sulfonylhydrazon-, Hydrazon- bzw. Iminderivaten erfolgt dabei durch im Stand der Technik bekannte Verfahren.

Es ist weiterhin möglich, eine HO-Gruppe oder eine NH₂-Gruppe einer pharmazeutisch und/oder diagnostisch aktiven Substanz in eine Carbonylkomponente zu überführen, beispielsweise duch eine Veresterung bzw. Amidbildung mit einer Carbonsäue-tragenden Carbonylkomponente gemäß den folgenden allgemeinen Reaktionsschemata, 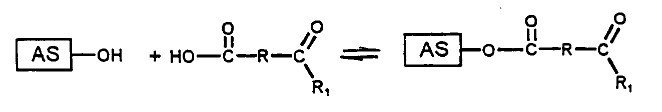 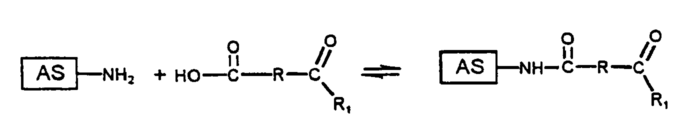 wobei R eine aliphatische Kohlenstoffkette und/oder ein aliphatischer Kohlenstoffring und/oder ein Aromat und R₁ = H, Alkyl, eine unsubstituierte Phenylgruppe oder ein substituierter Phenylrest ist. R besteht vorzugsweise aus 1 bis 12 Kohlenstoffatomen, die gegebenenfalls substituiert, beispielsweise durch hydrophile Gruppen wie Sulfonsäure-, Aminoalkyl- oder Hydroxygruppen, sein können. Der Aromat ist vorzugsweise ein Benzolring, der gegebenenfalls substituiert sein kann. Bevorzugte Substituenten sind beispielsweise die vorstehend genannten hydrophilen Gruppen.

Die Carbonylkomponente kann des weiteren durch andere chemische Reaktionen eingeführt werden, beispielsweise durch eine elektrophile Substitution an der HO- oder NH₂-Gruppe des Wirkstoffs mit einer geeigneten Carbonylkomponente.

Die derart derivatisierten Pharmaka, die nunmer eine Carbonylkomponente aufweisen, werden analog zu den vorstehend beschriebenen Verfahren mit den Trägermolekül-bindenden Spacermolekülen, die eine Amino-, Hydrazid- oder Hydrazingruppe aufweisen, zu den entsprechenden Carboxylhydrazon-, Sulfonylhydrazon-, Hydrazon- bzw. Iminderivaten umgesetzt. Die Spaltung dieser säurelabilen Bindungen führt demnach zu einer Freisetzung der derivatisierten pharmazeutisch und/oder diagnostisch aktiven Substanz, die eine Carbonylkomponente aufweist.

Die Gruppen, die aus der thiolbindenden Gruppe und dem Spacermolekül bestehen, können beispielsweise gemäß Verfahren, die u.a. in DE-A-196 36 889, U. Beyer et al., 1997 (Chemical Monthly, 128, 91, 1997), R.S. Greenfield et al., 1990 (Cancer Res., 50, 6600, 1990), T. Kaneko et al., 1991 (Bioconjugate Chem., 2, 133, 1991), Bioconjugate Techniques (G.T. Hermanson, Academic Press, 1996) oder im US-Patent 4,251,445 beschrieben sind, hergestellt werden.

Pharmaka oder Pharmakaderivate der erfindungsgemäßen Konjugate, die eine Peptidbindung enthalten, können beispielsweise dadurch hergestellt werden, daß ein Peptid, das aus 2 bis etwa 30 Aminosäuren besteht, mit einer thiolbindenden Verbindung umgesetzt wird, so daß eine thiolbindende Gruppe direkt oder über ein Spacermolekül am N-terminalen Ende des Peptids eingeführt wird. Die Synthese von solchen Trägermolekül-bindenden Peptidderivaten erfolgt vorzugsweise durch eine einem Fachmann bekannte Festphasensynthese, wobei im letzten Schritt des Peptidaufbaus ein Carbonsäure-tragendes, Trägermolekülbindendes Spacermolekül, z.B. eine Maleinimidcarbonsäure, durch Peptidkopplung an das N-terminale Ende der Peptids gebunden und das Trägermolekül-bindende Peptid anschließend von der Festphase abgespalten wird.

Die so erhaltenen Peptidderivate können mit Pharmaka oder Pharmakaderivaten, die eine H₂N- oder HO-Gruppe besitzen, in der Gegenwart eines Kondensationsmittels, wie zum Beispiel N,N'-Dicyclohexylcarbodiimid (DCC) oder N-Cyclohexyl-N'-(2-morpholinoethyl)-carbodiimid-metho-p-toluol-sulfonat (CMC) oder (Benzotriazol-1-yloxy)-trispyrrolidinophosphoniumhexafluorophosphat (pyBOP) oder Benzotriazol-N,N,N',N'-tetramethyluroniumhexafluorophosphat, und gegebenenfalls unter Zusatz von N-Hydroxysuccinimid oder eines wasserlöslichen N-Hydroxysuccinimids, wie etwa des Natriumsalzes der N-Hydroxysuccinimid-3-sulfonsäure, oder 1-Hydroxybenzotriazol und/oder in Gegenwart einer Base, beispielsweise N-Methylmorpholin oder Triethylamin, zu den entsprechenden thiolbindenden Pharmaka-Peptidderivaten umgesetzt werden:

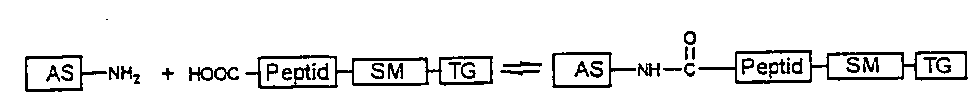

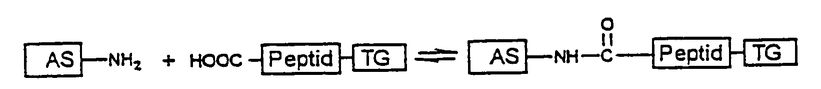

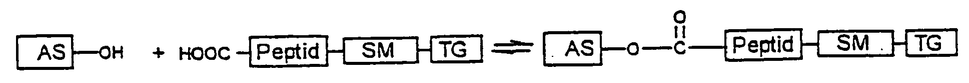

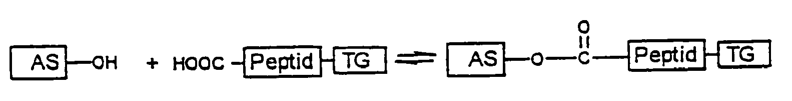

Es ist weiterhin möglich, über die HOOC-Gruppe der Pharmaka der erfindungsgemäßen Konjugate eine H₂N- oder HO-Gruppe einzuführen, beispielsweise durch Derivatisierung über die α-Aminogruppe der Aminosäuren Lysin, Serin oder Threonin oder mit einer Diaminoverbindung der allgemeinen Formel H₂N-(CH₂)ₙ-NH₂ oder einem Alkoholamin der allgemeinen Formel H₂N- (CH₂)ₙ-OH mit n = 1 bis 12, und diese Derivate im Anschluß mit den oben genannten Peptidderivaten zu den entsprechenden thiolbindenden Pharmaka-Peptidderivaten umzusetzen:

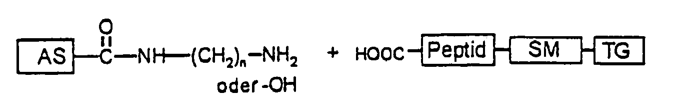

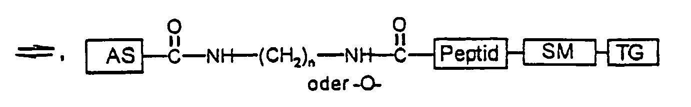

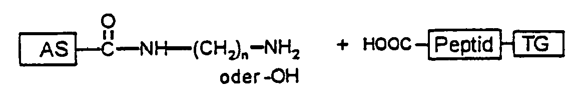

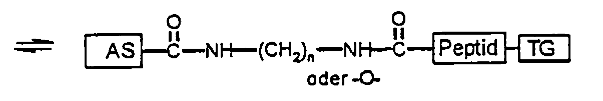

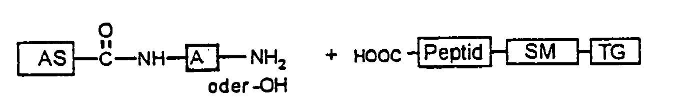

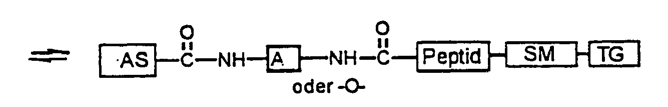

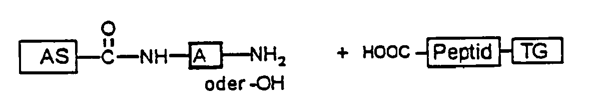

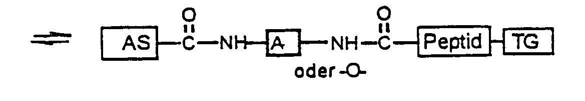

### A = Lysin, Serin oderThreonin

Die Substratspezifität von Targetenzymen, wie etwa von MMP 2, MMP 3, MMP 9, Cathepsin B und H, ist bekannt (Netzel-Arnett et al. (1993), Biochemistry 32, 6427-6432, Shuja, S., Sheahan, K., Murname, M.J. (1991), Int.J. Cancer 49, 341-346, Lah, T.T., Kos, J. (1998), Biol. Chem. 379, 125-130).

Beispielsweise sind Octapeptide (P₄- P'₄) für MMP 2 und MMP 9 (siehe Tabelle 1) identifiziert worden, welche die Spaltsequenz der Kollagenkette simulieren, und besonders effizient von MMP 2 und 9 gespalten werden (Aminosäuren sind im folgenden entsprechend dem internationalen Dreibuchstabencode abgekürzt):

**Tabelle 1:**

| Peptid | | | | | | | |
|---|---|---|---|---|---|---|---|
| P₄ | P₃ | P₂ | P₁ | P'₁ | P'₂ | P'₃ | P'₄ |
| Gly-Pro-Leu-Gly-Ile-Ala-Gly-Gln | | | | | | | |
| Gly-Pro-Gln-Gly-Ile-Trp-Gly-Gln | | | | | | | |

(Netzel-Arnett et al., Biochemistry 32, 1993, 6427-6432)

Die Peptide werden ausschließlich an der P₁ - P'₁-Bindung enzymatisch gespalten.

Desweiteren sind bei Cathepsin B substratspezifische Peptide bekannt mit der Sequenz -Gly-Phe-Leu-Gly-, -Gly-Phe-Ala-Leu-, -Ala-Leu-Ala-Leu-, -Arg-Arg-oder -Phe-Lys- (Werle, B., Ebert, E., Klein, W., Spiess, E. (1995), Biol. Chem. Hoppe-Seyler 376, 157-164; Ulricht, B., Spiess, E., Schwartz-Albiez, R., Ebert, W. (1995), Biol. Chem. Hoppe-Seyler 376, 404-414).

Die Peptidsequenz, welche die für das Targetenzym relevante Peptidsollbruchstelle enthält, kann auch so aufgebaut sein, daß die Peptidsollbruchstelle mehrfach wiederholt wird, wie beispielsweise durch:
-Gly-Pro-Leu-Gly- Ile-Ala-Gly-Gln-Gly-Pro-Leu-Gly- Ile-Ala-Gly-Gln
   oder
-Phe-Lys-Phe-Lys-Phe-Lys-Phe-Lys-Phe-Lys-Phe-Lys-
   oder es kann eine repetitive Peptidsequenz integriert werden, die den Abstand zwischen der thiolbindenden Gruppe und der relevanten Peptidsollbruchstelle vergrößert, wie beispielsweise durch:
-(Gly)ₙ-Phe-Lys-Phe-Lys-
mit vorzugsweise n = 2 bis 20, mehr bevorzugt n ≤ 12.

Ein wichtiges Merkmal dieser Ausführungsform des erfindungsgemäßen Konjugats ist die Tatsache, daß die für das jeweilige Targetenzym relevante Peptidsollbruchstelle mindestens einmal in einem Oligopeptid, bestehend aus etwa 1 bis 30 Aminosäuren, vorkommt. Die oben aufgeführten Oligopeptide sind repräsentative Beispiele für die enzymatisch spaltbare Bindung in den erfindungsgemäßen Konjugaten und schränken die Erfindung nicht ein.

Pharmaka oder Pharmakaderivate der erfindungsgemäßen Konjugate, die ein Zytokin enthalten, können beispielsweise dadurch hergestellt werden, daß das Zytokin mit einem eine thiolbindende Gruppe enthaltenen Spacermolekül, das eine Carbonsäure oder eine aktivierte Carbonsäure aufweist, umgesetzt wird:

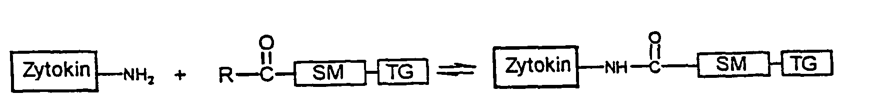

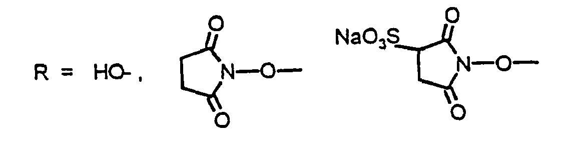

Weist das Spacermolekül eine N-Hydroxysuccinimidester-Gruppe (N-Hydroxysuccinimid oder N-Hydroxysuccinimid-3-sulfonsäure, Natriumsalz) auf, wird es direkt mit dem Zytokin umgesetzt. Die Umsetzung des Zytokins mit einem eine thiolbindende Gruppe enthaltenen Spacermolekül, das eine Carbonsäure aufweist, erfolgt in der Gegenwart eines Kondensationsmittels, wie zum Beispiel N,N -Dicyclohexylcarbodiimid (DCC) oderN-Cyclohexyl-N'-(2-morpholinoethyl)-carbodiimid-metho-p-toluolsulfonat (CMC), und gegebenenfalls unter Zusatz von N-Hydroxysuccinimid oder dem Natriumsalz der N-Hydroxysuccinimid-3-sulfonsäure, zu den entsprechenden thiolbindenden Zytokinderivaten. Die Aufreinigung der so derivatisierten Zytokine erfolgt in der Regel mit Hilfe der Ausschlußchromatographie. Die oben beschriebenen Umsetzungen sind einem Fachmann geläufig (siehe z.B. Bioconjugate Techniques, G.T. Hermanson, Academic Press, 1996).

Die oben beschriebenen Pharmaka oder Pharmakaderivate werden an natives oder rekombinantes Albumin als Träger gekoppelt, so daß im erfindungsgemäßen Konjugat pro Mol Cystein-34-Rest mehr als 0,7 Mol, vorzugsweise mindestens 0,9 Mol, Pharmakon über die thiolbindende Gruppe an Albumin gebunden sind. Im Idealfall sind im erfindungsgemäßen Konjugat daher 100% der im Albumin vorhandenen Cystein-34-Reste über die thiolbindende Gruppe mit einem Pharmakon verbunden.

Eine weitere Ausführungsform der vorliegenden Erfindung betrifft somit ein Verfahren zur Herstellung eines wie oben definierten Konjugats, umfassend
(i) Behandlung des Albumins mit einem Reduktionsmittel, so daß mehr als 0,7 Mol, vorzugsweise mindestens 0,9 Mol, Cystein-SH-Gruppen pro Mol Cystein-34-Rest von Albumin vorliegen, wobei die Behandlung in einen pH-Bereich von 5,0 bis 8,0 bei einem Verhältnis von Reduktionsmittel zu Träger zwischen 0,5:1 und 10:1 und einer Reaktionszeit zwischen 1h und 96h durchgeführt wird, und
(ii) Kopplung des Pharmakons über die thiolbindende Gruppe an die Cystein-34-SH-Gruppen von Albumin.

In einer bervorzugten Ausführungsform des erfindungsgemäßen Verfahrens ist das zur Behandlung des Trägers verwendete Reduktionsmittel Dithiothreitol (DTT), Dithioerythritol (DTE) oder Mercaptoethanol. Das besonders bevorzugte Reduktionsmittel ist DTT.

Das erfindungsgemäße Verfahren beruht auf der Erkenntnis, daß die im Stand der Technik bekannten Träger in einem inhomogenen Oxidationsstatus vorliegen. Beispielsweise lassen sich im Fall des im Handel erhältlichen nativen Albumins in der Regel mit dem photometrischen Essay nach Ellmann ≈ 0,2 bis 0,7 Mol HS-Gruppen pro Mol Cystein-Reste im Albumin nachweisen, d.h. das Cystein-34 ist häufig durch schwefelhaltige Verbindungen, wie etwa Cystein oder Glutathion, über eine Disulfidbindung oxidiert. Das bedeutet, daß die im Albumin vorhandenen Cystein-SH-Gruppen zumindest häufig nicht frei vorliegen, was bisher dazu führte, daß die Ausbeute an hergestellten Konjugaten zu gering und/oder stark schwankend von Albumin- zu Albumincharge war.

Die Reaktion wird vorzugsweise so gesteuert, daß mindestens 0,9 Mol Cystein-34-SH-Gruppen pro Mol Cystein-34-Rest von Albumin verfügbar werden.

Die Umsetzung des Reduktionsmittels mit einem im Handel erhältlichen Albumin erfolgt,beispielsweise in einem Salzpuffer, z.B. in 0,01 M Natriumborat, 0,15 M NaCl, 0,001 M EDTA oder 0,15 M NaCl, 0,004 M Phosphat in einem pH-Bereich von 5,0 bis 8,0, vorzugweise von 6,0 bis 7,0.

Das Verhältnis von Reduktionsmittel zu Albumin beträgt zwischen 0,5:1 und 10:1. Die Reaktionszeit beträgt zwischen 1 h und 96h, vorzugsweise zwischen 6h und 24h.

Das mit dem Reduktionsmittel behandelte Albumin wird z.B. durch Gelfiltration (beispielsweise Sephadex® G10 oder G25, Laufmittel: 0,004 M Phosphat, 0,15 M NaCl - pH 7,4) oder durch Ultrafiltration isoliert.

Die Konzentration an Albumin nach erfolgter Gelfiltration wird durch den Extinktionskoeffizienten bei 280 nm, die Anzahl der eingeführten HS-Gruppen wird mit Ellmann's Reagenz bei 412 nm bestimmt. Die so isolierte Trägerlösung kann direkt für die Synthese der Konjugate eingesetzt werden. Es ist auch möglich, die Trägerlösung mit einem handelsüblichen Konzentrator aufzukonzentrieren oder zu lyophilisieren. Die isolierte Trägerlösung oder das Lyophilisat kann im Temperaturbereich von -78 bis +30 °C gelagert werden.

Die Kopplung der oben beschriebenen Pharmakaderivate an Albumin erfolgt beispielsweise bei Raumtemperatur. Dabei wird Albumin, das sich in einem Salzpuffer (beispielsweise 0,15 M NaCl - pH 6,0 bis 8,0), der eventuell vorher entgast wurde, befindet, ein etwa 1,1- bis 10-facher Überschuß des wie oben beschrieben hergestellten Pharmakons (bezogen auf die Cystein-34-HS-Gruppen von Albumin), gelöst in einer minimalen Menge Lösungsmittel, beispielsweise DMF, Dimethylsulfoxid, Wasser, Salzpuffer, Ethanol, Methanol, Propylenglykol, Glycerin, Acetonitril oder THF (etwa 1 bis 10% des Volumens der Albuminprobe), gegeben. Es ist auch möglich, das Pharmakon als Festsubstanz zur Albumin lösung zu geben. Desweiteren kann es vorteilhaft sein, vor der Kopplung einen Hilfsstoff, wie etwa eine Fettsäure oder ein Tryptophonatderivat, zur Albumin lösung zu geben. Nach einer Reaktionszeit zwischen 5 min und 48 h wird die Lösung, falls erforderlich, zentrifugiert, und das gebildete Albumin-Pharmakon-Konjugat wird durch anschließende Gelfiltration (beispielsweise Sephadex® G10 oder G25) in einem Salzpuffer, wie etwa 0,004 M Phosphat, 0,15 M NaCl - pH 6,0 bis 8,0, isoliert.

Die Reinheit des entstandenen Konjugats kann beispielsweise durch HPLC, z.B. durch Außschlußchromatographie, überprüft werden. Im Gegensatz zu herkömmlichen Konjugaten weisen die gemäß einer bevorzugten Ausführungsform des erfindungsgemäßen Verfahren hergestellten Konjugate eine Reinheit von mehr als 95% auf.

Die Lösung des so erhaltenen Konjugats kann mit einem handelsüblichen Konzentrator aufkonzentriert werden. Die Konjugate können in gelöster Form bei + 1 bis + 30 °C oder in gefrorener Form bei T = 0 °C bis -78 °C gelagert werden. Desweiteren ist es möglich, die Lösung der Konjugate zu lyophilisieren und das Lyophilisat bei +30° bis -78 °C zu lagern.

Eine weitere Ausführungsform der vorliegenden Erfindung betrifft ein Arzneimittel, enthaltend ein wie oben definiertes Konjugat, und gegebenenfalls einen pharmazeutisch verträglichen Träger und/oder Hilfsstoff und/oder ein Verdünnungsmittel. Das erfindungsgemäße Arzneimittel kann bevorzugt zur Behandlung von Krebskrankheiten, Autoimmunerkrankungen, akuten oder chronisch-entzündlichen Erkrankungen und Erkrankungen, die durch Viren oder Mikroorganismen wie beispielsweise Bakterien und/oder Pilze verursacht sind, verwendet werden.

Noch eine weitere Ausführungsform der vorliegenden Erfindung betrifft einen diagnostischen Kit, enthaltend ein wie oben definiertes Konjugat. Der erfindungsgemäße diagnostische Kit kann bevorzugt zum Nachweis der wie vorstehend definierten Erkrankungen und/oder zum Nachweis von Albuminmolekülen und/oder deren Verteilung im Körper verwendet werden.

Die Figuren zeigen:

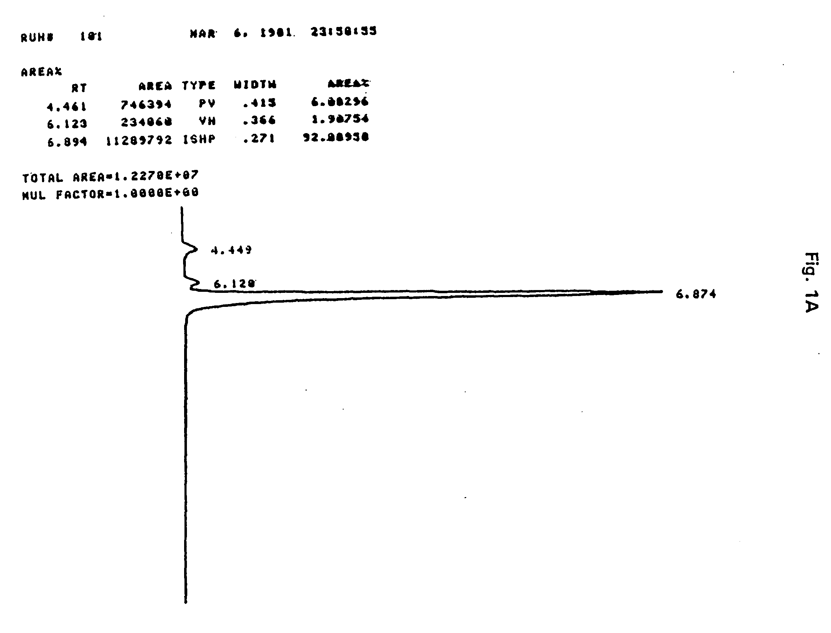

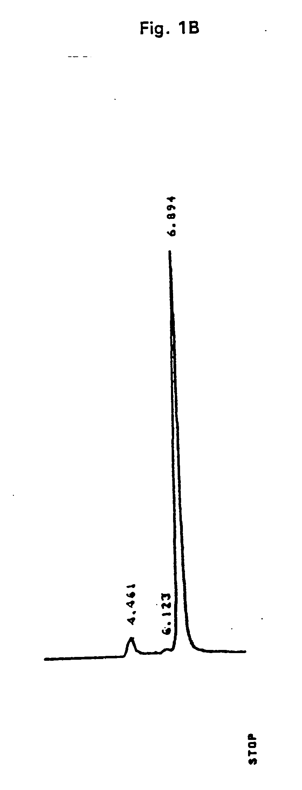

- Fig. 1: (A) ist ein HPLC-Chromatogramm eines erfindungsgemäßen Konjugats (A-DOXO-HYD-C). Es ist die Absorption bei 495 nm gegen die Retentionszeit in min aufgetragen. (B) ist das entsprechende HPLC-Chromatogramm von im Handel erhältlichem nativem Albumin (lmmuno GmbH).

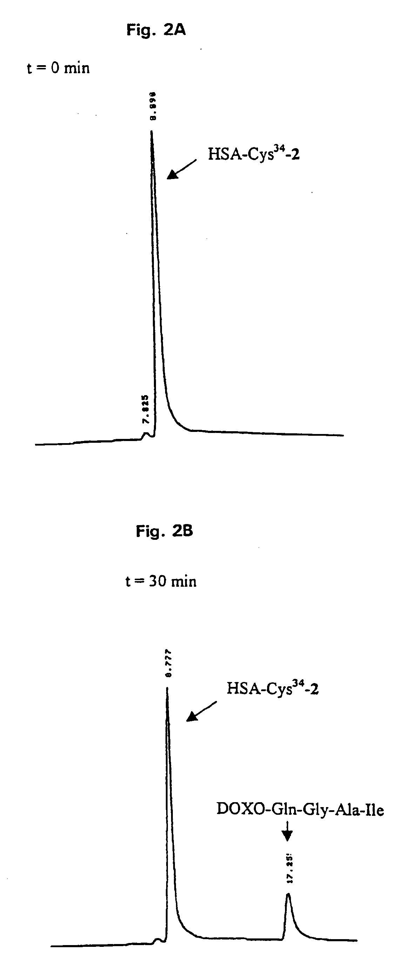

- Fig. 2: zeigt HPLC-Chromatogramme (Ausschlußchromatographie-Säule Biosil 250 SEC der Fa. Biorad) eines erfindungsgemäßen Konjugats (HSA-Cys³⁴-2), welches durch die Matrixmetalloprotease MMP 9 spaltbar ist. Es ist jeweils die Absorption bei 495 nm gegen die Retentionszeit in min aufgetragen. (A) Chromatogramm des Konjugats HSA-Cys³⁴-2 vor der Inkubation mit MMP 9 (t=0). (B) Chromatogramm des Konjugats HSA-Cys³⁴-2 nach der Inkubation mit MMP 9 für 30 min (t=30 min).

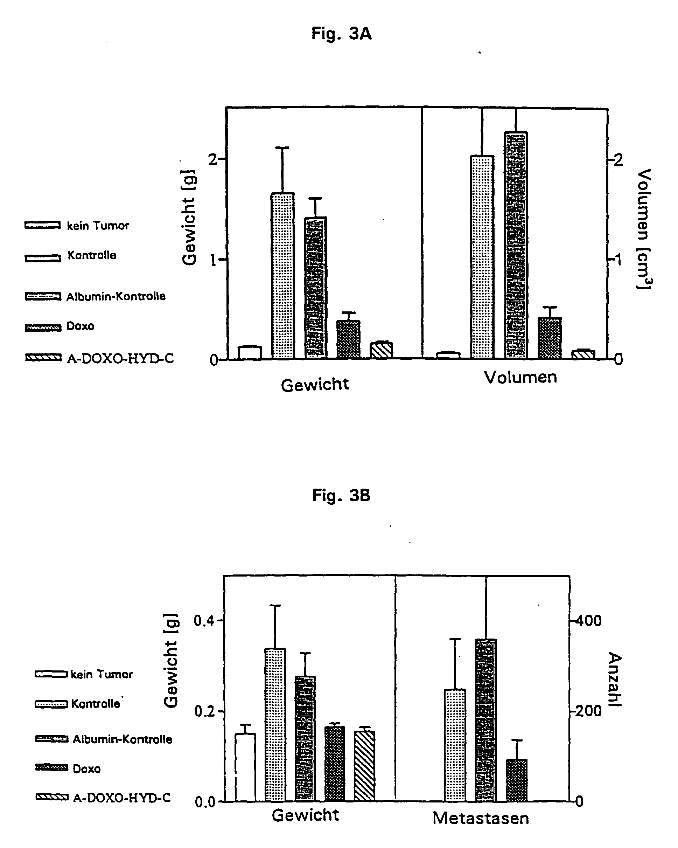

- Fig. 3: zeigt die graphische Darstellung der Gewichte und Volumen von Nieren und Nierentumoren (A) sowie der Gewichte von Lungen und die Anzahl der Lungenmetastasen (B) von Mäusen, bei denen ein Nierenkarzinom erzeugt wurde, und die den angebenen Behandlungen ausgesetzt wurden (Kontrolle: keine Behandlung; Albumin-Kontrolle: natives Albumin; Doxo: Doxorubicin; A-DOXO-HYD-C: erfindungsgemäßes Konjugat). Zum Vergleich sind auch die Daten für Mäuse, denen keine Tumorzellen injiziert wurden, abgebildet (kein Tumor).

Das folgende Beispiel erläutert die vorliegende Erfindung näher, ohne sie einzuschränken.

### BEISPIEL

Umsetzung von humanem Serumalbumin (HSA) mit Dithiothreitol (DTT)

Das Verfahren für die Behandlung von HSA mit einem Reduktionsmittel wird durch folgendes Beispiel genauer dargestellt: 2,0 g humanes Serumalbumin (10 ml einer 20%igen HSA-Lösung, Pharma Dessau) wird mit 10 ml Puffer A (0,004 M Natriumphosphat, 0,15 M NaCl - pH 7,0) verdünnt und mit 100 *µ*l einer frisch hergestellten 0,036 x 10⁻² M DTT-Lösung (5,55 mg DTT gelöst in 100 *µ*l Puffer A) versetzt und das Reaktionsgefäß sanft unter Luftausschluss während 16 h bei Raumptemperatur geschüttelt. Anschließend wird die Albuminlösung durch Gelfiltration (Säule 5,0 cm x 25,0 cm, Sephadex® G.25; Laufpuffer 0,004 M Natriumphosphat, 0,15 M NaCl - pH 7,4) aufgereinigt. Die Proteinkonzentration nach erfolgter Gelfiltration wurde photometrisch bei 280 nm (ε(HSA)₂₈₀ = 35 700 M⁻¹ cm⁻¹ - c[HSA] ≈ 3,1 x 10⁻⁴ M) und die Anzahl der eingeführten HS-Gruppen mit Ellmanns Reagenz bei 412 nm (ε₄₁₂ = 13 600 M⁻¹ cm⁻¹ - c[HS-Gruppen] = 3,07 x 10⁻⁴ M) bestimmt. Daher liegen im so behandelten HSA 0,99 Mol freie Cystein-SH-Gruppen pro Mol Cystein-Rest vor. Das behandelte HSA wurde auf etwa 1,0 x 10⁻³ M eingeengt (Centriprep-10^{®}) und direkt für die unten aufgeführte Kopplungsreaktion mit einem thiolbindenden Pharmakon der vorliegenden Erfindung verwendet.

### Herstellung des erfindungsgemäßen Konjugats A-DOXO-HYD-C

Das HSA-Doxorubicin-Konjugat (A-DOXO-HYD-C), bestehend aus gemäß obigem Beispiel mit DTT behandeltem HSA und einem Maleinimidophenylessigsäurehydrazon-Derivat von Doxorubicin (DOXO-HYD), wurde weiterhin folgendermassen hergestellt.

### Struktur von DOXO-HYD:

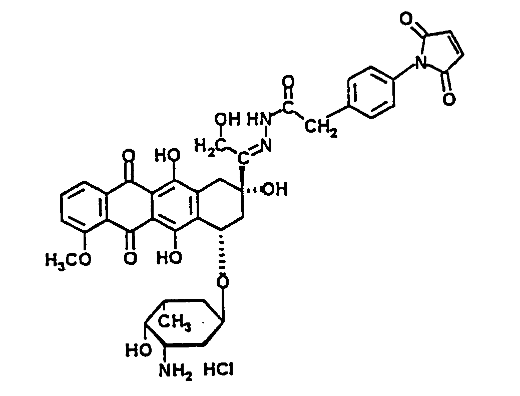

12 ml der mit DTT behandelten HSA-Probe (Sulfhydrylgehalt von 0,99 Mol pro Mol HSA) wurden mit 0,6 ml einer Lösung von DOXO-HYD (Mr 807,8) in DMF (12,5 mg gelöst in 0,6 ml DMF) versetzt und die Reaktionslösung während 18 h sanft geschüttelt. Das entstandene HSA-Doxorubicin-Konjugat wurde über eine Sephadex® G-25F Säule (Säule 5,0 cm x 25 cm) isoliert (Retentionsvolumen: 85 - 135 ml). Die Menge an gebundenem Doxorubicin wurde mit Hilfe des Extinktionskoeffizienten von Doxorubicin bei 495 nm (ε₄₉₅ = 10 650 M⁻¹ cm⁻¹ bei pH 7,4) bestimmt. Danach sind in diesem Beispiel pro Mol Cystein-Rest im HSA 0,97 Mol Doxorubicin an das HSA gebunden.

*Methoden-* FPLC für die Herstellung der Konjugate: P-500 pump, LCC 501 Controller (Pharmacia) und LKB 2151 UV-Monitor. Die Proteinkonzentration des Konjugats wurde photometrisch sowie mit dem BCA-Protein-Essay von Pierce (USA) bestimmt.

Die Reinheit des Konjugats A-DOXO-HYD-C wurde durch HPLC mit Hilfe einer analytischen Säule (Bio-Sil SEC 250, (300 mm x 7.8 mm) von Bio-RAD (mobile Phase: i.d.R. 0.15 M NaCl, 0.01 M NaH₂PO₄, 5% CH₃CN - pH 7.0) bei λ = 495 nm geprüft. Die HPLC-Chromatogramme für A-DOXO-HYD-C und von im Handel erhältlichem nativem Albumin (lmmuno GmbH) sind in der Fig. 1 A (A-DOXO-HYD-C) und der Fig. 1 B (natives Albumin) abgebildet. Es ist deutlich zu erkennen, daß A-DOXO-HYD-C eine dem im Handel erhältlichen nativen Albumin vergleichbare, hervorragende Reinheit aufweist. 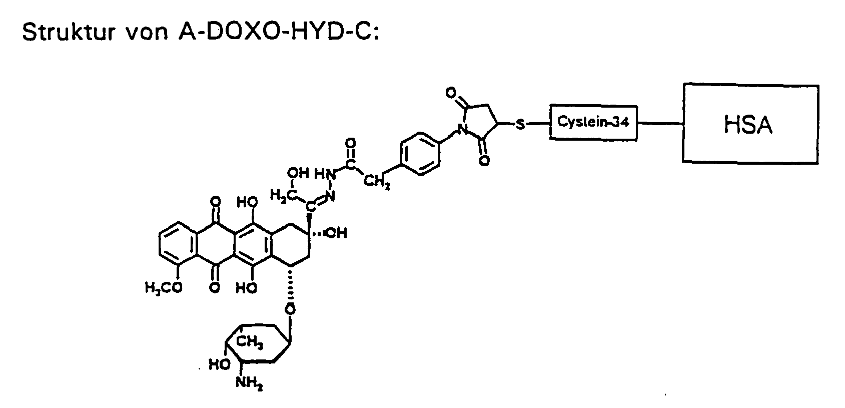 (HSA = humanes Serumalbumin)

**Herstellung eines erfindungsgemäßen Konjugats, bestehend aus mit DTT behandeltem HSA und einem durch MMP 9 spaltbaren Doxorubicin-Maleinimid-Peptid-Derivat**

Das Doxorubicin-Maleinimid-Peptid-Derivat (2) wurde gemäß folgender Reaktionsgleichung hergestellt.

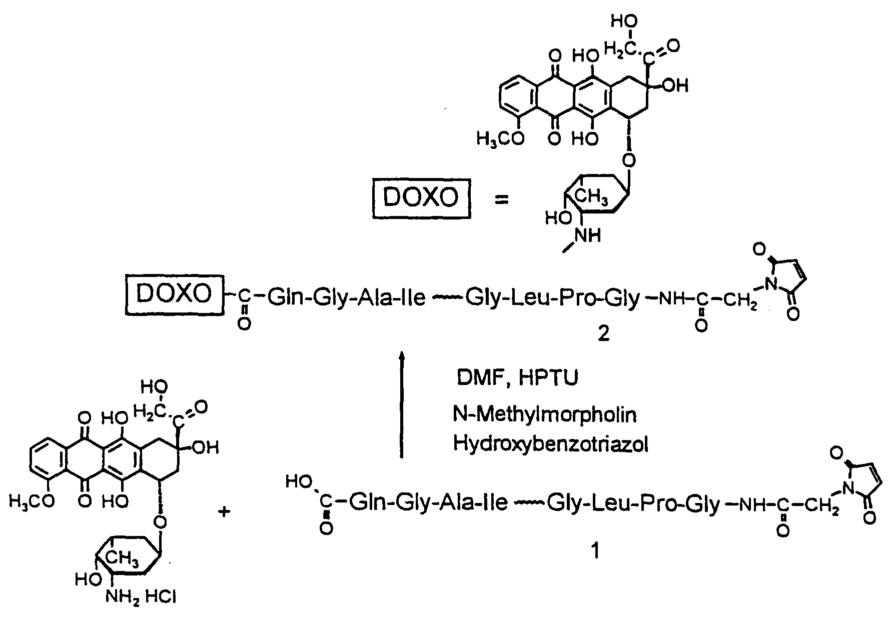

Dabei wurde das mit Maleinimidoglycin derivatisierte Octapeptid Gln-Gly-Ala-Ile-Gly-Leu-Pro-Gly 1 (Mr 848, hergestellt durch Festphasensynthese durch Bachem AG, Schweiz) mit Doxorubicin gemäß dem folgenden Verfahren umgesetzt:

Zu einer leicht trüben Lösung von 17,1 mg Doxorubicin in 3 ml DMF werden 25 mg 1 (als Trifluoracetatsalz), gelöst in 500 µl DMF, 33,5 mg O-Benzotriazol-N,N,N',N'-tetramethyluroniumhexafluorophosphat (HPTU), gelöst in 200 µl DMF, 11,9 mg Hydroxybenzotriazolhydrat, gelöst in 100 µl DMF, und 16,2 µl N-Methylmorpholin zugegeben und der Ansatz anschließend 18 h lang bei RT in der Dunkelheit gerührt. DMF wurde unter Hochvakuum entfernt und der Feststoff in 20 ml Methanol aufgenommen, filtriert und im Vakuum auf 1 ml eingeengt. Nach Aufreinigung über Kieselgel (Essigester/Methanol 2/1) wurden 5 mg 2 erhalten.

3,0 ml einer mit DTT behandelten HSA-Probe (Sulfhydrylgehalt von 0,95 pro HSA-Molekül, Gehalt an HS-Gruppen ~ 1000 µM) wurden mit einer Lösung von 2 (Mr 1374) in DMF (5,1 mg, gelöst in 250 µl DMF) versetzt und die Reaktionslösung während 30 min sanft geschüttelt. Das entstandene Albumin-Doxorubicin-Konjugat wurde über eine Sephacryl® HR100-Säule (2,0 cm x 20 cm) isoliert. Auf diese Weise wurde das Albuminkonjugat (im folgenden mit HSA-Cys³⁴-2 bezeichnet) der folgenden Struktur isoliert (Beladungsfaktor ~0,9):

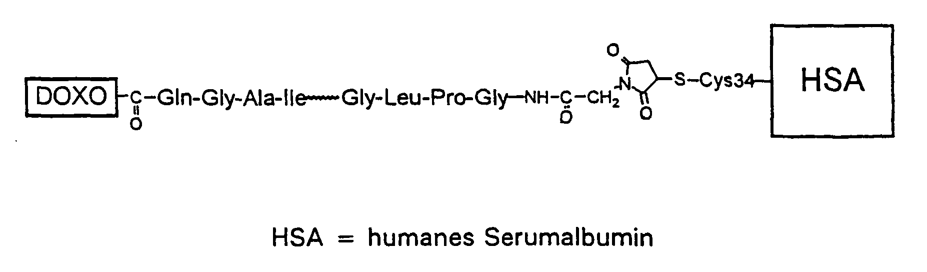

Die Peptidsequenz Gln-Gly-Ala-Ile-Gly-Leu-Pro-Gly wird von der Matrixmetalloprotease MMP 9 erkannt und zwischen Isoleucin und Glycin gespalten. Dies wurde durch folgenden Versuch gezeigt: 200 µl einer 100 µM Lösung von HSA-Cys³⁴-2 wurde mit Trypsin/Aprotinin-aktivierter MMP 9 (2 mU, erhalten von Calbiochem, Deutschland) 30 Minuten lang bei 37°C inkubiert. Die Freisetzung von DOXO-Gln-Gly-Ala-Ile aufgrund der Spaltung mit MMP 9 wurde durch HPLC-Ausschlußchromatographie (Biosil 250 SEC Säule der Fa. Biorad, Detektion bei λ = 495 nm) vor der Inkubation (t = 0, vgl. Fig. 2A) und nach einer Inkubationszeit mit aktivierter MMP 9 von 30 Minuten (t=30, vgl. Fig. 2B) bestätigt.

### Biologische Untersuchungen

Als Beispiel für die *in vivo* Wirksamkeit der erfindungsgemäßen Konjugate werden die biologischen Daten der HSA-Doxorubicin-Konjugats A-DOXO-HYD-C aufgeführt.

Im sogenannten RENCA (renal cell carcinoma)-Modell wurden Doxorubicin und das erfindungsgemäße Konjugat A-DOXO-HXD-C hinsichtlich der antitumoralen Wirksamkeit bei annähernd äquitoxischer Dosis miteinander verglichen (intravenöse Therapie 10 Tage nach Injektion von etwa 1 Million Nierenkarzinomzellen in die linke Niere).

**Tiere: Balb/c-** Maüse, **weiblich; Tumor:** RENCA, renal cell carcinoma **Therapie:** Tag(d) 10, 14, 18, 21 intravenös (i.v.), Ende des Versuchs: d 25

Die Ergebnisse dieser Untersuchungen sind in der Tabelle 2 zusammengefaßt.

**Tabelle 2**

| Anzahl der Mäuse | Substanz | Dosis (mg/Kg/inj.) | Mortalität (d) | Durchschnittliche Körpergewichtsabnahme (%) d 1 bis 25 |
|---|---|---|---|---|
| 10 | Kontrolle | | 2 | - 14 |
| 10 | Albumin-Kontrolle | 4x1,4 g | 1 | - 16 |
| 10 | Doxorubicin (Doxo) | 4x6 mg/kg | 1 | - 21 |
| 10 | A-DOXO-HYD-C | 4x12 mg/kg | 0 | - 18 |

Die Dosis bezieht sich auf die vorhandene Menge Doxorubicin. Die Dosierungen von Doxorubicin und A-DOXO-HYD-C sind annähernd äquitoxisch (siehe Körpergewichtsabnahme in der Tabelle 2).

Die Ergebnisse dieses Versuches sind desweiteren in der Fig. 3 hinsichtlich der Gewichte und Volumina der Nieren und Nierentumoren (Fig. 3A) sowie der Gewichte der Lungen und der Anzahl der Lungenmetastasen (Fig. 3B) graphisch dargestellt. A-DOXO-HYD-C zeigt eine sehr gute antitumorale Wirksamkeit und erzielt eine komplette Remission in allen Tieren. Makroskopisch sichtbare Lungenmetastasen konnten nur in einem Tier beobachtet werden (Fig. 3B). Bei der mit Doxorubicin behandelten Gruppe wurden deutlich sichtbare Nierentumoren in allen Tieren beobachtet (Fig. 3A), d.h. bei der optimalen Dosis von Doxorubicin (Körpergewichtabnahme: -21 % (d 1 bis 25); 1 Tier verstorben) konnten demgegenüber keine kompletten Remissionen erzielt werden. Weiterhin betrug bei den mit freiem Doxorubicin behandelten Mäusen die Anzahl der Lungenmetastasen im Durchschnitt etwa 100 Metastasen pro Maus (Fig. 3B).

## Patentansprüche

1. Arzneimittel, enthaltend ein Träger-Pharmakon-Konjugat, und gegebenenfalls einen pharmazeutisch verträglichen Träger und/oder einen Hilfsstoff und/oder ein Verdünnungsmittel, **dadurch gekennzeichnet, dass**
(i) der Träger natives oder rekombinantes Albumin ist,
(ii) das Pharmakon eine pharmazeutisch und/oder diagnostisch aktive Substanz ist,
(iii) das Pharmakon über ein Spacermolekül und eine thiolbindende Gruppe an Cystein-34 des Albumins gebunden ist,
(iv) das Spacermolekül und/oder die Verknüpfung zwischen der pharmazeutisch und/oder diagnostisch aktiven Substanz und dem Spacermolekül und/oder die Verknüpfung zwischen der thiolbindenden Gruppe und dem Spacermolekül hydrolytisch und/oder pH-abhängig und/oder enzymatisch spaltbar ist, und
(v) mehr als 0,7 Mol Pharmakon pro Mol Albumin an Cystein-34 gebunden ist.

2. Arzneimittel nach Anspruch 1, wobei das Spacermolekül und/oder die Verknüpfung mindestens eine Peptidbindung enthält.

3. Arzneimittel nach Anspruch 2, wobei die Peptidbindung innerhalb einer Peptidsequenz vorliegt, welche mindestens eine Spaltsequenz einer Protease enthält.

4. Arzneimittel nach einem der Ansprüche 1 bis 3, wobei das Spacermolekül und/oder die Verknüpfung mindestens eine säurelabile Bindung enthält.

5. Arzneimittel nach einem der Ansprüche 1 bis 4, wobei die pharmazeutisch aktive Substanz ein Zytostatikum, ein Zytokin, ein Immunsuppressivum, ein Antirheumatikum, ein Antiphlogistikum, ein Antibiotikum, ein Analgetikum, ein Virostatikum oder ein Antimykotikum ist.

6. Arzneimittel nach Anspruch 5, wobei das Zytostatikum aus der Gruppe der Anthrazykline, der N-Nitrosoharnstoffe, der Alkylantien, der Purin- oder Pyrimidinantagonisten, der Folsäureantagonisten, der Taxane, der Camptothecine, der Podophyllotoxinderivate, der Vinca-Alkaloide, der Calicheamicine, der Maytansinoide oder der cis-konfigurierten Platin(II)-Komplexe ausgewählt ist.

7. Arzneimittel nach einem der Ansprüche 1 bis 6, wobei die diagnostisch wirksame Substanz ein oder mehrere Radionuklide, ein oder mehrere Radionuklide umfassende Liganden, ein oder mehrere Positronenstrahler, ein oder mehrere NMR-Kontrastmittel, eine oder mehrere fluoreszierende Verbindung(en) oder ein oder mehrere Kontrastmittel im nahen IR-Bereich enthält.

8. Arzneimittel nach einem der Ansprüche 1 bis 7, wobei die thiolbindende Gruppe eine Maleinimidgruppe, eine Halogenacetamidgruppe, eine Halogenacetatgruppe, eine Pyridyldithio-Gruppe, eine Vinylcarbonylgruppe, eine Aziridingruppe, eine Disulfidgruppe oder eine Acetylengruppe umfaßt, die gegebenenfalls substituiert sind.

9. Arzneimittel nach einem der Ansprüche 1 bis 8, wobei das Spacermolekül einen substituierten oder unsubstituierten, verzweigt- oder unverzweigtkettigen aliphatischen Alkylrest mit 1 bis 12 Kohlenstoffatomen und/oder mindestens einen substituierten oder unsubstituierten Arylrest und/oder einen aliphatischen Kohlenstoffring mit 3 bis 12 Kohlenstoffatomen umfaßt.

10. Verfahren zur Herstellung des in dem Arzneimittel nach einem der Ansprüche 1 bis 9 enthaltenen Konjugats, umfassend
(i) Behandlung des Albumins mit einem Reduktionsmittel, so daß mehr als 0,7 Mol, Cystein-34-SH-Gruppen pro Mol Cystein-34-Rest von Albumin vorliegen, wobei die Behandlung in einem pH-Bereich von 5,0 bis 8,0, bei einem Verhältnis von Reduktionsmittel zu Albumin zwischen 0,5:1 und 10:1 und einer Reaktionszeit zwischen 1 h und 96 h durchgeführt wird, und
(ii) Kopplung des Pharmakons über die thiolbindende Gruppe an die Cystein-34-SH-Gruppen von Albumin.

11. Verfahren nach Anspruch 10, wobei das Reduktionsmittel Dithiothreitol, Dithioerythritol oder Mercaptoethanol ist.

12. Verfahren nach Anspruch 10 oder 11, wobei das hergestellte Konjugat eine Reinheit von mehr als 95% aufweist.

13. Arzneimittel nach einem der Ansprüche 1 bis 9 zur Behandlung von Krebskrankheiten, Autoimmunkrankheiten, akuten oder chronisch-entzündlichen Erkrankungen und Erkrankungen, die durch Viren und/oder Mikroorganismen verursacht werden.

14. Diagnostischer Kit, enthaltend das Arzneimittel nach einem der Ansprüche 1 bis 9.

15. Kit nach Anspruch 14 zum Nachweis von Krebskrankheiten, Autoimmunkrankheiten, akuten oder chronisch-entzündlichen Erkrankungen und Erkrankungen, die durch Viren und/oder Mikroorganismen verursacht werden, und/oder von Molekülen des Albumins und/oder deren Verteilung im Körper.

## Claims

1. Medicament comprising a carrier-drug conjugate, and where appropriate a pharmaceutically acceptable carrier and/or an excipient and/or a diluent,
**characterized in that**
(i) the carrier is native or recombinant albumin,
(ii) the drug is a pharmaceutically and/or diagnostically active substance,
(iii) the drug is linked via a spacer molecule and a thiol-binding group to cysteine 34 of the albumin,
(iv) the spacer molecule and/or the linkage between the pharmaceutically and/or the diagnostically active substance and the spacer molecule and/or the linkage between the thiol-binding group and the spacer molecule can be cleaved by hydrolysis and/or pH-dependently and/or enzymatically, and
(v) more than 0.7 mole of drug is linked to cysteine 34 per mole of albumin.

2. Medicament according to Claim 1, where the spacer molecule and/or the linkage comprises at least one peptide bond.

3. Medicament according to Claim 2, where the peptide bond is present inside a peptide sequence which comprises at least one protease cleavage sequence.

4. Medicament according to any of Claims 1 to 3, where the spacer molecule and/or the linkage comprises at least one acid-labile bond.

5. Medicament according to any of Claims 1 to 4, where the pharmaceutically active substance is a cytostatic, a cytokine, an immunosuppressant, an antirheumatic, an anti-inflammatory drug, an antibiotic, an analgesic, a virostatic or an antimycotic.

6. Medicament according to Claim 5, where the cytostatic is selected from the group of anthracyclins, of N-nitrosoureas, of alkylating agents, of purine or pyrimidine antagonists, of folic acid antagonists, of taxanes, of camptothecins, of podophyllotoxin derivatives, of vinca alkaloids, of calicheamycins, of maytansinoids or of cis-configuration platinum(II) complexes.

7. Medicament according to any of Claims 1 to 6, where the diagnostically active substance comprises one or more radionuclides, one or more ligands including radionuclides, one or more positron emitters, one or more NMR contrast media, one or more fluorescent compound(s) or one or more contrast media in the near IR region.

8. Medicament according to any of Claims 1 to 7, where the thiol-binding group includes a maleimide group, a haloacetamide group, a haloacetate group, a pyridyldithio group, a vinylcarbonyl group, an aziridine group, a disulphide group or an acetylene group, which are optionally substituted.

9. Medicament according to any of Claims 1 to 8, where the spacer molecule includes a substituted or unsubstituted, branched- or unbranched-chain aliphatic alkyl radical having 1 to 12 carbon atoms and/or at least one substituted or unsubstituted aryl radical and/or an aliphatic carbon ring having 3 to 12 carbon atoms.

10. Method for preparing the conjugate present in the medicament according to any of Claims 1 to 9, comprising
(i) treating the albumin with a reducing agent so that more than 0.7 mole of cysteine 34 SH groups are present per mole of cysteine 34 residue of albumin, where the treatment is carried out in a pH range from 5.0 to 8.0, with a ratio of reducing agent to albumin of between 0.5:1 and 10:1 and with a reaction time of between 1 h and 96 h, and
(ii) coupling the drug via the thiol-binding group to the cysteine 34 SH groups of albumin.

11. Method according to Claim 10, where the reducing agent is dithiothreitol, dithioerythritol or mercaptoethanol.

12. Method according to Claim 10 or 11, where the prepared conjugate has a purity of more than 95%.

13. Medicament according to any of Claims 1 to 9 for the treatment of cancers, autoimmune diseases, acute or chronic inflammatory disorders and disorders caused by viruses and/or microorganisms.

14. Diagnostic kit comprising the medicament according to any of Claims 1 to 9.

15. Kit according to Claim 14 for the detection of cancers, autoimmune diseases, acute or chronic inflammatory disorders and disorders caused by viruses and/or microorganisms, and/or of molecules of albumin and/or distribution thereof in the body.

## Revendications

1. Médicament contenant un conjugué excipient-produit pharmaceutique, et éventuellement un excipient pharmaceutiquement acceptable et/ou un composé auxiliaire et/ou un diluant, **caractérisé en ce que**
(i) l'excipient est de l'albumine native ou recombinante,
(ii) le produit pharmaceutique est une substance active pharmaceutiquement et/ou à des fins de diagnostic,
(iii) le produit pharmaceutique est lié à la cystéine 34 de l'albumine au moyen d'une molécule espaceuse et d'un groupe pouvant se lier à un thiol,
(iv) la molécule espaceuse et/ou la liaison entre la substance active pharmaceutiquement et/ou à des fins de diagnostic et la molécule espaceuse et/ou la liaison entre le groupe pouvant se lier à un thiol et la molécule espaceuse pouvant être coupées de manière hydrolytique et/ou de manière dépendante du pH et/ou de manière enzymatique, et
(v) plus de 0,7 mol de produit pharmaceutique par mol d'albumine est lié à la cystéine 34.

2. Médicament selon la revendication 1, la molécule espaceuse et/ou la liaison contenant au moins une liaison peptidique.

3. Médicament selon la revendication 2, la liaison peptidique étant présente à l'intérieur d'une séquence peptidique, laquelle contient au moins une séquence d'une protéase pouvant être coupée.

4. Médicament selon l'une quelconque des revendications 1 à 3, la molécule espaceuse et/ou la liaison contenant au moins une liaison stable en milieu acide.

5. Médicament selon l'une quelconque des revendications 1 à 4, la substance pharmaceutiquement active étant un cytostatique, une cytokine, un immunosuppresseur, un antirhumatismal, un anti-inflammatoire, un antibiotique, un analgésique, un virostatique ou un antimycosique.

6. Médicament selon la revendication 5, le cytostatique étant choisi parmi le groupe constitué des anthracyclines, des N-nitrosourées, des alkylantiènes, des antagonistes de purines ou de pyrimidines, des antagonistes de l'acide folique, des taxanes, des camptothécines, des dérivés de podophyllotoxine, des vinca-alcaloïdes, des calichéamicines, des maytansinoïdes ou des complexes de platine(II) en configuration cis.

7. Médicament selon l'une quelconque des revendications 1 à 6, la substance active à des fins de diagnostic contenant un ou plusieurs ligands comportant des radionucléides, un ou plusieurs émetteurs de positrons, un ou plusieurs agents de contraste en RMN, un ou plusieurs composé(s) fluorescent(s) ou un ou plusieurs agents de contraste dans le domaine du proche IR.

8. Médicament selon l'une quelconque des revendications 1 à 7, le groupe pouvant se lier à un thiol comportant un groupe maléimide, un groupe halogénoacétamide, un groupe halogénoacétate, un groupe pyridyldithio, un groupe vinylcarbonyle, un groupe aziridine, un groupe disulfure ou un groupe acétylène, lesquels sont éventuellement substitués.

9. Médicament selon l'une quelconque des revendications 1 à 8, la molécule espaceuse comportant un radical alkyle aliphatique substitué ou non susbtitué à chaîne ramifiée ou non ramifiée ayant 1 à 12 atomes de carbone et/ou au moins un radical alkyle substitué ou non substitué et/ou un cycle carboné aliphatique ayant 3 à 12 atomes de carbone.

10. Procédé de fabrication du conjugué contenu dans le médicament selon l'une quelconque des revendications 1 à 9, comportant
(i) le traitement de l'albumine avec un milieu réducteur, de sorte que plus de 0,7 mol de groupes SH de la cystéine 34 par mol de radicaux cystéine 34 de l'albumine soient présents, le traitement étant effectué dans un domaine de pH de 5,0 à 8,0, dans une proportion du milieu réducteur par rapport à l'albumine entre 0,5:1 1 et 10:1 et un temps de réaction entre 1 h et 96 h, et
(ii) le couplage du produit pharmaceutique au moyen du groupe pouvant se lier à un thiol au groupe SH de la cystéine 34 de l'albumine.

11. Procédé selon la revendication 10, le milieu réducteur étant du dithiothréitol, du dithioérythritol ou du mercaptoéthanol.

12. Procédé selon la revendication 10 ou 11, le conjugué fabriqué présentant une pureté de plus de 95 %.

13. Médicament selon l'une quelconque des revendications 1 à 9 destiné au traitement de maladies cancéreuses, de maladies auto-immunes, d'affections inflammatoires aigües ou chroniques et d'affections, qui sont provoquées par des virus et/ou des microorganismes.

14. Kit de diagnostic, contenant le médicament selon l'une quelconque des revendications 1 à 9.

15. Kit selon la revendication 14 pour la détection de maladies cancéreuses, de maladies auto-immunes, d'affections inflammatoires aigües ou chroniques et d'affections, qui sont provoquées par des virus et/ou des microorganismes, et/ou de molécules d'albumine et/ou de leur distribution dans l'organisme.
